# FASCICULE DE BREVET EUROPEEN

(11) **EP 1 441 791 B1**
(45) Date de publication et mention de la délivrance du brevet: **29.06.2011**
(21) Numéro de dépôt: 02788034.3
(22) Date de dépôt: 05.11.2002
(51) Int. Cl.: A61M 16/12

(54) **DISPOSITIF POUR ASSISTANCE RESPIRATOIRE**
VORRICHTUNG ZUR UNTERSTÜTZUNG DER ATMUNG
RESPIRATORY ASSISTANCE DEVICE

(30) Priorité: 06.11.2001 FR 0114318
(43) Date de publication de la demande: 04.08.2004
(73) Titulaire: Boussignac, Georges, 92160 Antony (FR)
(72) Inventeur: Boussignac, Georges, 92160 Antony (FR)
(74) Mandataire: Bonnetat, Christian
(86) Numéro de dépôt international: PCT/FR2002/003779
(87) Numéro de publication internationale: WO 2003/039638

(56) Documents cités:
- GB-A- 550 725
- US-A- 3 913 607
- US-A- 5 538 002

## Description

La présente invention a pour objet un dispositif pour assistance respiratoire, utilisable sur des patients dont la respiration spontanée est absente ou insuffisante, qu'ils soient placés ou non sous respiration artificielle.

On connaît divers dispositifs, tels que des masques, des sondes ou des canules orales, nasales, endotrachéales, trachéotomiques, destinés à faire la jonction entre un appareil de respiration artificielle et/ou d'anesthésie et le système respiratoire d'un patient. Ces dispositifs, essentiellement en forme de tubes, peuvent, selon le cas, comporter des moyens d'immobilisation tels que des pattes ou des collerettes au voisinage de l'extrémité proximale, pour le maintien sur la bouche ou le nez du patient, ou encore des ballonnets gonflables au voisinage de l'extrémité distale, pour le maintien par friction dans la trachée.

Les dispositifs connus présentent des inconvénients importants. Ainsi, par exemple, lorsqu'un tube de type connu est déconnecté du respirateur artificiel et que le patient a besoin d'air enrichi en oxygène, il est nécessaire d'introduire dans ledit tube une sonde reliée à une source d'oxygène. Par ailleurs, dans les cas de respiration spontanée insuffisante, le patient doit nécessairement rester relié au respirateur jusqu'au rétablissement complet de sa respiration spontanée.

-Aussi, pour-remédier à ces inconvénients, on a déjà proposé, par exemple dans les documents EP-A-0 390 684, EP-A-0 701 834 et EP-A-0 978 291, des dispositifs d'assistance respiratoire qui, outre le canal principal formé par le tube, comportent au moins un canal auxiliaire, par exemple ménagé dans la paroi dudit tube, permettant l'injection d'un jet de gaz respirable destiné à la ventilation du patient, ce canal auxiliaire débouchant dans le canal principal en avant de l'extrémité distale de ce dernier.

Dans ces dispositifs, le gaz respirable alimentant le canal auxiliaire est, le plus souvent, de l'oxygène pur. Or, certains patients, dont l'organisme est habitué à un taux élevé de gaz carbonique dans le sang, ne peuvent supporter une ventilation à l'oxygène pur, qui risquerait d'entraîner un malaise cardiaque.

L'objet de la présente invention est de perfectionner les dispositifs d'assistance respiratoire rappelés ci-dessus pour leur permettre de prendre en compte le cas de ces derniers patients.

A cette fin, selon l'invention, le dispositif d'assistance respiratoire comportant un tube qui forme un canal principal et qui est destiné à être relié par sa portion distale à une voie respiratoire d'un patient pour que ledit canal principal relie, à l'extérieur, le système respiratoire dudit patient, ledit dispositif comportant de plus au moins un canal auxiliaire relié à une source de gaz respirable pour pouvoir insuffler un jet d'un tel gaz respirable dans ledit système respiratoire et débouchant dans ledit canal principal par au moins un orifice disposé en avant de l'extrémité distale de ce dernier, est remarquable en ce qu'il comporte, entre ledit orifice dudit canal auxiliaire et ladite extrémité distale dudit canal principal, des moyens de communication commandables à l'ouverture et à la fermeture et aptes, lorsqu'ils sont en position ouverte, à former un passage qui relie ledit canal principal à l'ambiance extérieure et à travers lequel de l'air extérieur est aspiré par ledit jet de gaz respirable du fait de la zone de dépression engendrée à l'intérieur dudit canal principal par lesdits moyens de déflexion.

Ainsi, grâce-à-la-présente invention, lorsque lesdits moyens de communication sont ouverts, de l'air extérieur est aspiré à travers eux par ledit jet de gaz respirable, l'air ainsi introduit diluant ledit gaz respirable, qui peut alors être toléré par les patients, dont le cas a été rappelé ci-dessus.

De préférence, afin que le dispositif puisse être adapté à toutes les circonstances et à tous les patients, il est avantageux que ledit passage reliant ledit canal principal à l'ambiance ait une section variable. Ainsi, il est possible d'ajuster de façon optimale la dilution du gaz respirable par l'air ambiant.

Dans un mode de réalisation pratique desdits moyens de communication, ceux-ci sont du type à bague tournante percée latéralement et apte à découvrir des passages de diamètres différents. Une telle bague peut être montée directement sur ledit tube ou bien sur une cheminée en communication avec ledit canal principal.

Il est avantageux que, en regard de l'orifice distal dudit canal auxiliaire, soient prévus des moyens de déflexion du jet de gaz respirable de ventilation vers l'axe dudit canal et que lesdits moyens de communication soient disposés entre lesdits moyens de déflexion et ladite extrémité distale du canal principal. En effet, dans ce cas, lesdits moyens de déflexion créent, dans ledit canal principal, une dépression favorable à l'aspiration de l'air ambiant à travers lesdits moyens de communication.

Les figures du dessin annexé feront bien comprendre comment l'invention peut être réalisée. Sur ces figures, des références identiques désignent des éléments semblables.
La figure 1 est une vue schématique et partielle, en coupe axiale agrandie, d'un mode de réalisation du dispositif de l'invention.
Les figures 2, 3 et 4 sont des coupes transversales, respectivement-selon-les lignes-II-II, III-III et IV-IV de la figure-1-.
La figure 5 montre, en vue semblable à la figure 1, une variante de réalisation du dispositif selon l'invention.

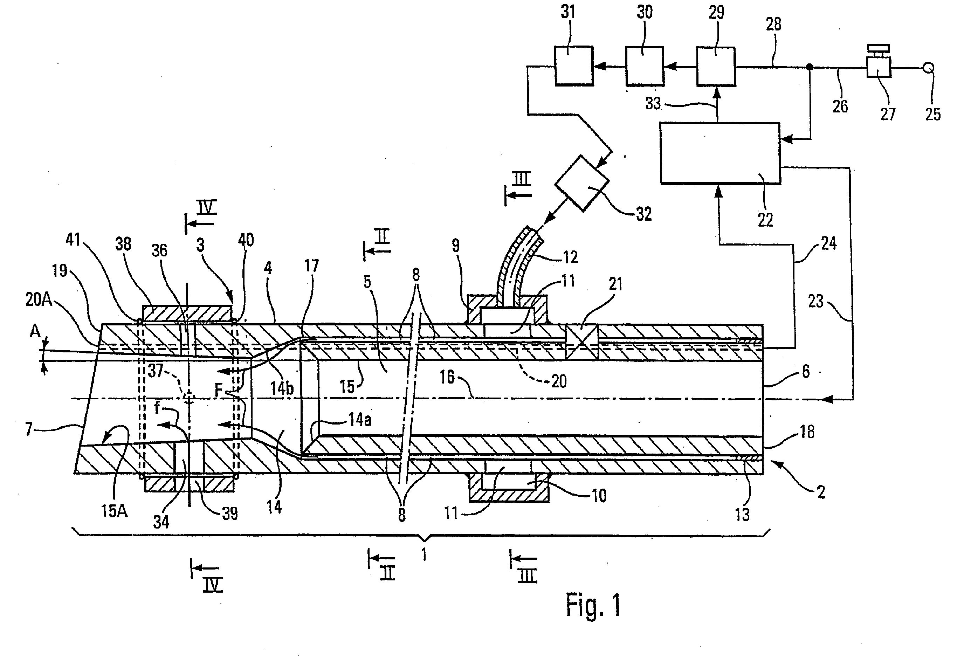

Sur la figure 1, on a représenté, schématiquement et à grande échelle, les seules portions proximale 2 et distale 3 d'un mode de réalisation 1 du dispositif selon l'invention. Ce mode de réalisation peut constituer, par exemple, une sonde endotrachéale oro-nasale avec ou sans ballonnet, une sonde endotrachéale pédiatrique, une sonde de monitorage des gaz, une sonde endobronchique, une sonde naso-pharyngée, une sonde d'intubation anatomique pour enfant, une sonde de Cole néonatale, une sonde canule de Gedel, une sonde nasale d'oxygénothérapie, un masque nasal ou bucconasal ou un ballon nasal pour traitement d'apnée du sommeil.

Le dispositif 1 comporte un tube 4, souple ou préformé (pour s'adapter à la morphologie du patient) délimitant un canal principal 5 ayant un orifice proximal 6 et un orifice distal 7, respectivement aux extémités dudit tube.

Ainsi, le canal principal 5 est capable d'assurer le passage entre les orifices 6 et 7, dont l'un (l'orifice 7) est destiné à se trouver à l'intérieur des voies respiratoires d'un patient, et l'autre (l'orifice 6) est destiné à se trouver à l'extérieur dudit patient. Cet orifice 6 peut déboucher à l'air libre et, dans ce cas, le patient peut inspirer de l'air frais et expirer l'air vicié à travers le canal principal 5, On peut également, comme cela est expliqué ci-après, relier l'orifice 6 à une source de gaz respirable sous pression et prévoir un système de valves unidirectionnelles, pour que le patient inspire le gaz respirable de ladite source à travers ledit canal principal 5 et expire le gaz vicié à l'air libre, également à travers ce canal principal.

Le diamètre du canal principal 5 est de l'ordre de quelques millimètres. Des essais satisfaisants ont été effectués avec des-diamètres-de-3-mm, 7 mm, 8 mm et 12 mm.

Par ailleurs, dans l'épaisseur de la paroi du tube 4, sont ménagés des canaux auxiliaires 8, s'étendant sur la presque totalité de la longueur du canal principal et destinés à être reliés à une source de gaz respirable sous pression, comme cela est décrit ci-après.

La liaison à la source de gaz respirable sous pression peut être réalisée au moyen d'une bague 9, entourant de façon étanche le tube 4, du côté de l'extrémité proximale 2, et délimitant une chambre annulaire étanche 10 autour dudit tube. Les canaux auxiliaires 8 sont mis en communication avec la chambre 10, grâce à des arrachements locaux 11 de la paroi du tube 4, et ladite chambre 10 est reliée à ladite source de gaz respirable par un conduit 12. Bien entendu, les extrémités proximales des canaux 8 sont obturées, par exemple par des bouchons 13, introduits à partir de la face d'extrémité proximale 18 du tube 4.

Les canaux auxiliaires 8 ont un diamètre plus petit que celui du canal principal 5. Le diamètre des canaux auxiliaires 8 est de préférence inférieur à 1 mm et, de façon avantageuse, il est de l'ordre de 400 à 800 microns. Du côté distal, les canaux auxiliaires 8 débouchent dans un évidement 14 de la paroi interne 15 du tube 4. L'évidement 14 est annulaire et centré sur l'axe 16 dudit tube. Il comporte une face 14a, sensiblement transversale ou légèrement inclinée de façon à constituer un évasement du canal principal 5, dans laquelle débouchent lesdits canaux auxiliaires 8 par leurs orifices 17, ainsi qu'une face 14b suivant la face 14a et convergeant en direction de l'axe 16.

De préférence, entre la face inclinée convergente 14b et l'orifice distal 7, la paroi interne 15 présente une partie 15A légèrement évasée vers l'extérieur, comme cela est illustré par l'angle A sur la figure 1.

Ainsi, lorsque les canaux auxiliaires 8 sont alimentés en gaz respirable sous pression à travers les éléments 9 à 12, les jets gazeux correspondants heurtent la face inclinée 14b, qui les défléchit en direction de l'axe 16 (flèches F sur la figure 1), engendrant au voisinage de celui-ci une zone de dépression favorisant la circulation gazeuse à l'intérieur du canal principal 5, de l'orifice proximal vers l'orifice distal. On favorise ainsi l'inspiration du patient.

De préférence, la distance entre chacun des orifices 17 et l'orifice 7 est de l'ordre de 1 à 2 cm.

Au moins un canal supplémentaire 20 est prévu dans l'épaisseur du tube 4 afin de déboucher en 20A au voisinage de l'extrémité distale 19 du tube 4 et servir de prise de pression.

A titre de sécurité, une soupape d'échappement tarée 21 peut être prévue dans l'extrémité proximale2 du tube 4. Ainsi, en cas de surpression accidentelle dans le canal principal 5, une fuite de gaz se produit à l'extérieur du patient, à travers la paroi du tube 4, pour éliminer instantanément cette surpression.

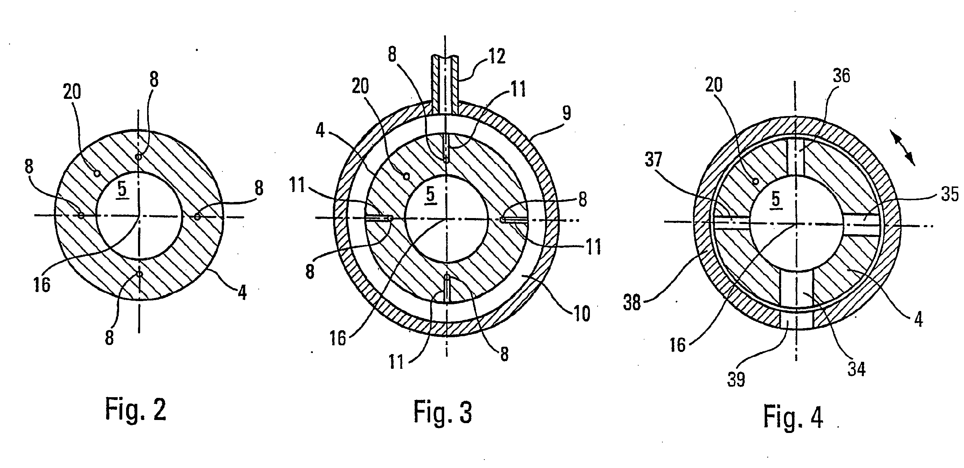

Comme le montrent les figures 2 et 3, les canaux auxiliaires 8 sont disposés régulièrement autour de l'axe du tube 4. Leur nombre est variable suivant les utilisations (adulte ou enfant), mais il est généralement compris entre trois et neuf. De plus, un au moins des canaux auxiliaires 8 peut être spécialisé pour apporter un fluide médical.

Le tube 4 du dispositif selon l'invention peut être réalisé en toute matière déjà utilisée dans les sondes respiratoires, par exemple en un chlorure de polyvinyle, avec un éventuel revêtement de silicone ou en acier permettant les injections à pression élevée.

Bien entendu, les dimensions du dispositif selon l'invention peuvent être très variables, essentiellement en fonction de la voie de mise en place du tube et de la taille du patient, qui peut être un adulte, un enfant, un nourrisson ou un prématuré.

Le dispositif 1 comporte de plus un dispositif d'alimentation et de commande 22, qui est respectivement relié à l'orifice 6 de l'extrémité proximale 2 du tube 4 par une liaison 23 et au canal supplémentaire 20 par une liaison 24.

Le dispositif d'alimentation et de commande 22 est alimenté en gaz respirable sous pression, par exemple de l'oxygène pur, par une source 25, à laquelle il est relié par une conduite 26 sur laquelle est monté un détendeur-débitmètre réglable 27.

La sortie du détendeur-débitmètre 27 est reliée au conduit 12 par une conduite de dérivation 28 sur laquelle sont montés en série une vanne commandable 29, un dispositif à perte de charge réglable limiteur de débit et de pression 30 (par exemple un tube à conduit calibré), un humidificateur 31 et une soupape d'échappement tarée 32, à tarage réglable. La vanne commandable 29 est commandée par le dispositif d'alimentation et de commande 22 par l'intermédiaire d'une liaison 33.

A titre d'exemple non limitatif, le détendeur-débitmètre 27 peut délivrer, dans la conduite 28, le gaz respirable provenant de la source 25 sous une pression P, par exemple égale à 3,5 bars avec un débit maximal réglable de par exemple 32 litres par minute, alors que le limiteur de débit et de pression 30, recevant ce gaz respirable de la conduite 28, peut en abaisser la pression jusqu'à une valeur p, égale par exemple à 0,5 bar (pour un adulte) et à 0,07 bar (pour un enfant), et le débit jusqu'à une valeur d égale, par exemple, à 0,5 litre par minute. Quant à elle, la soupape d'échappement 32 est tarée à la pression p.

Par ailleurs (voir les figures 1 et 4), entre l'évidement annulaire 14 et l'orifice distal 7, la paroi du tube 4 est percée par des trous transversaux 34 à 37, de diamètres différents et répartis autour de l'axe 16. Les trous 34 à 37 sont recouverts par une bague 38, apte à tourner à frottement doux autour dudit tube 4 et elle-même pourvue d'un trou 39 pouvant être-amené en regard de l'un ou l'autre des trous 34 à 37, par rotation de la bague 38. Le trou 39 a un diamètre au moins égal à celui du trou 34, qui est le plus grand des trous 34 à 37. La bague 38 est prisonnière du tube 4, grâce à des nervures latérales annulaires 40 et 41.

Comme on peut le voir sur la figure 4, la bague 38 peut prendre soit au moins une position pour laquelle elle obture tous les trous 34 à 37, soit des positions pour lesquelles le trou 39 est aligné avec chacun des trous 34 à 37, respectivement. Dans ces derniers cas, à chaque fois, un passage est établi entre le canal principal 5 et l'ambiance extérieure, à travers le trou 34 à 37 correspondant. Bien entendu, la section d'un tel passage est alors déterminée par la section du trou 34 à 37 considéré.

Les modes de fonctionnement du dispositif 1 selon l'invention sont les suivants :
- dans le mode de respiration artificielle, la bague 38 obture tous les trous 34 à 37 et le dispositif d'alimentation et de commande 22, d'une part, commande la vanne 29 à la fermeture par l'intermédiaire de la liaison 33, de sorte que le conduit 12 n'est pas alimenté en gaz et, d'autre part, adresse du gaz respirable dans le tube 4 par l'intermédiaire de la liaison 23. Ce dispositif 22 comporte des moyens (non représentés) pour permettre le réglage de la pression et du débit de gaz respirable qu'il reçoit de la conduite 26 et qu'il adresse au tube 4. Si une surpression se produit dans la voie respiratoire du patient, elle est détectée et transmise, par le canal supplémentaire 20 et la liaison 24, au dispositif 22 qui arrête son fonctionnement. De plus, si cette surpression dépasse le seuil de tarage de la soupape tarée 21 -par exemple du fait que le canal supplémentaire 20 est obstrué par des mucosités et n'a pu transmettre l'information de surpression au dispositif 22-- cette soupape 21 s'ouvre et le canal proximal 5 est mis à l'atmosphère ;
- dans le mode d'assistance respiratoire, le dispositif d'alimentation et de commande 22 coupe la liaison 23-pour-mettre l'orifice 6-en communication avec l'atmosphère et commande la vanne 29 par la liaison 33 pour que celle-ci adresse au patient un jet, continuel ou impulsionnel, de gaz respirable à travers le limiteur 30, l'humidificateur 31, la soupape d'échappement tarée 32 et les canaux auxiliaires 8. Par ailleurs, la bague 38 est tournée pour amener le trou 39 en regard de l'un des trous 34 à 37, de sorte qu'une communication est réalisée entre le canal principal 5 et l'ambiance extérieure, à l'aval de l'évidement annulaire 14, là où une zone de dépression est engendrée par les jets gazeux sortant des canaux auxiliaires 8. Par suite, de l'air extérieur est aspiré à travers ladite communication (voir la flèche f) et mélangé auxdits jets gazeux, qui est ainsi dilué. Bien entendu, le taux de dilution de ces jets gazeux dépend du trou 34 à 37 qui est passant. On peut remarquer que, pour des conditions d'injection desdits jets gazeux constantes, le taux de dilution correspondant à chacun des trous 34 à 37 peut être étalonné une fois pour toutes, de sorte que l'on peut délivrer à un patient le mélange air-gaz respirable le plus approprié à son cas en choisissant le trou 34 à 37 en regard duquel on amènera le trou 39 de la bague 38. Si une surpression se produit dans la voie respiratoire du patient, comme cela a été décrit ci-dessus, cette surpression est détectée et transmise par le canal supplémentaire 20, de sorte que le dispositif 22 ferme la vanne 29 et que la conduite 28 cesse d'adresser du gaz au patient. Si le canal supplémentaire 20 est obstrué, le dispositif 22 n'est pas averti de la surpression dans la voie respiratoire du patient et ne peut s'arrêter, mais cette surpression entraîne une augmentation de pression dans les canaux auxiliaires 8 et le conduit 12. Lorsque cette augmentation de pression atteint le seuil d'ouverture de la soupape de sécurité 32, celle-ci s'ouvre et le jet de gaz respirable n'est plus adressé au patient, mais au contraire est dérivé vers l'extérieur par ladite soupape-de sécurité 32. Ainsi, bien-que dans ce dernier cas la sécurité 20A, 20, 24, 22, 29 n'ait pu fonctionner, le jet de gaz respirable ne peut atteindre le système respiratoire du patient.

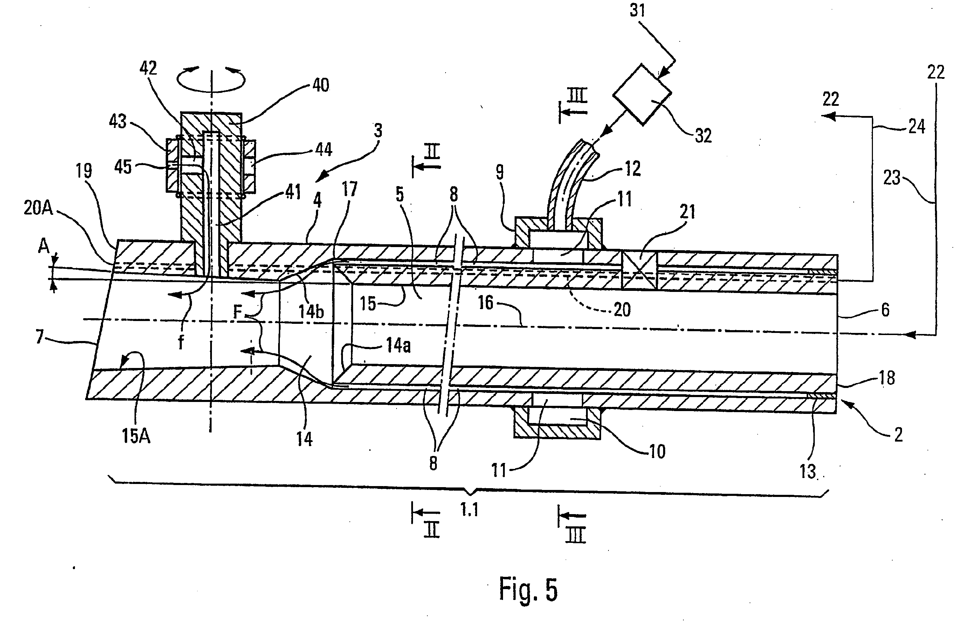

Dans la variante de réalisation 1.1 de la figure 5, le fonctionnement est identique à celui décrit ci-dessus. Dans cette variante de réalisation, on a prévu une cheminée borgne 40 en communication par un canal 41 avec le canal principal 5, ladite cheminée étant pourvue d'un trou latéral 42, recouvert par une bague tournante 43. Cette dernière bague est percée de trous 44, 45 de diamètres différents, au plus égaux à celui du trou 42, et elle peut, par rotation, soit obturer le trou 42, soit amener l'un des trous 44, 45 en regard du trou 42.

Ainsi, de ce qui précède, on voit qu'il est possible de diluer à l'air, dans toute proportion désirée, les jets de gaz provenant de la source 25 et passant à travers les canaux auxiliaires 8.

## Revendications

1. Dispositif d'assistance respiratoire comportant un tube (4) qui forme un canal principal (5) et qui est destiné à être relié par sa portion distale (3) à une voie respiratoire d'un patient pour que ledit canal principal (5) relie, à l'extérieur, le système respiratoire dudit patient, ledit dispositif comportant de plus au moins un canal auxiliaire (8) relié à une source de gaz respirable (25) pour pouvoir insuffler un jet d'un tel gaz respirable dans ledit système respiratoire et débouchant dans ledit canal principal (5) par au moins un orifice distal (17) disposé en avant de l'extrémité distale (7) de ce dernier, des moyens (14b) de déflexion dudit jet de gaz respirable de ventilation vers l'axe (16) dudit canal principal (5) étant prévus en regard dudit orifice distal (17) dudit canal auxiliaire (8),
**caractérisé en ce qu'**il comporte, entre lesdits moyens de déflexion (14b) et ladite extrémité distale (7) dudit canal principal (5), des moyens de communication (34-39 ; 40-45) commandables à l'ouverture et à la fermeture et aptes, lorsqu'ils sont en position ouverte, à former un passage
qui relie ledit canal principal (5) à l'ambiance extérieure et à travers lequel de l'air extérieur est aspiré par ledit jet de gaz respirable du fait de la zone de dépression engendrée à l'intérieur dudit canal principal (5) par lesdits moyens de déflexion (14b).

2. Dispositif selon la revendication 1,
**caractérisé en ce que** le passage reliant ledit canal principal (5) à l'ambiance extérieure a une section variable.

3. Dispositif selon la revendication 2,
**caractérisé en ce que** lesdits moyens de communication sont du type à bague tournante (38, 43) percée latéralement et apte à découvrir des passages de diamètres différents.

4. Dispositif selon la revendication 3,
**caractérisé en ce que** ladite bague tournante (38) est montée directement sur ledit tube (4).

5. Dispositif selon la revendication 3,
**caractérisé en ce que** ladite bague tournante (43) est montée sur une cheminée en communication avec ledit canal principal (5).

## Claims

1. A respiratory assistance device comprising a tube (4) which forms a main channel (5) and which is designed to be connected by its distal portion (3) to a respiratory airway of a patient such that said main channel (5) connects the respiratory system of said patient to the outside, said device further comprising at least one auxiliary channel (8) connected to a source (25) of breathable gas in order to be able to blow a stream of such a breathable gas into said respiratory system and emerging in said main channel (5) by at least one distal orifice (17) arranged in front of the distal end (7) of the latter, means (14b) of deflecting said ventilating breathable gas stream toward the axis (16) of said main channel (5) being provided facing said distal orifice (17) of said auxiliary channel (8),
**characterized in that** it comprises, between said deflecting means (14b) and said distal end (7) of said main channel (5), communication means (34-39; 40-45) which can be opened and closed and, when open, are capable of forming a passage which connects said main channel (5) to the external environment and through which external air is sucked by said breathable gas stream due to the zone of depression generated by said deflecting means (14b) inside said main channel (5).

2. The device as claimed in claim 1,
**characterized in that** the passage connecting said main channel (5) to the external environment has a variable cross section.

3. The device as claimed in claim 2,
**characterized in that** said communication means are of the type with a sideways-drilled rotary ring (38, 43) capable of uncovering passages of different diameters.

4. The device as claimed in claim 3,
**characterized in that** said rotary ring (38) is mounted directly on said tube (4).

5. The device as claimed in claim 3,
**characterized in that** said rotary ring (43) is mounted on a funnel in communication with said main channel (5).

## Patentansprüche

1. Vorrichtung zur Unterstützung der Atmung, die einen Tubus (4) aufweist, der einen Hauptkanal (5) bildet und dazu bestimmt ist, an seinem distalen Abschnitt (3) mit einem Atemweg eines Patienten verbunden zu werden, damit der Hauptkanal (5) das Atmungssystem des Patienten nach außen verbindet, wobei die Vorrichtung mindestens einen Hilfskanal (8) aufweist, der mit einer Quelle von Atemgas (25) verbunden ist, um einen Strom eines solchen Atemgases in das Atmungssystem einblasen zu können, und der über mindestens eine distale Öffnung (17) in den Hauptkanal (5) einmündet, die vor dem distalen Ende (7) des Letzteren angeordnet ist, Mittel (14b) zum Ablenken des Atemgas-Belüftungsstroms zur Achse (16) des Hauptkanals (5), die gegenüber der distalen Öffnung (17) des Hilfskanals (8) vorgesehen sind,
**dadurch gekennzeichnet, dass** sie zwischen den Mitteln zum Ablenken (14b) und dem distalen Ende (7) des Hauptkanals (5) Verbindungsmittel (34 - 39; 40 - 45) aufweist, die beim Öffnen und beim Schließen steuerbar und fähig sind, wenn sie sich in geöffneter Stellung befinden, einen Durchgang zu bilden, der den Hauptkanal (5) mit der Außenatmosphäre verbindet und durch den die Außenluft durch den Atemgasstrom angesaugt wird aufgrund der Unterdruckzone, die im Inneren des Hauptkanals (5) durch die Ablenkungsmittel (14b) erzeugt wird.

2. Vorrichtung nach Anspruch 1, **dadurch gekennzeichnet, dass** der Durchgang, der den Hauptkanal (5) mit der Außenatmosphäre verbindet, einen variablen Querschnitt besitzt.

3. Vorrichtung nach Anspruch 2, **dadurch gekennzeichnet, dass** die Verbindungsmittel dem Drehring-Typ (38, 43) entsprechen, der seitlich durchbohrt und fähig ist, Durchgänge mit verschiedenen Durchmessern freizugeben.

4. Vorrichtung nach Anspruch 3, **dadurch gekennzeichnet, dass** der Drehring (38) direkt auf dem Tubus (4) angebracht ist.

5. Vorrichtung nach Anspruch 3, **dadurch gekennzeichnet, dass** der Drehring (43) auf einem mit dem Hauptkanal (5) in Verbindung stehenden Rohr angebracht ist.
